# EUROPEAN PATENT SPECIFICATION

(11) **EP 2 775 934 B1**
(45) Date of publication and mention of the grant of the patent: **30.10.2019**
(21) Application number: 12844746.3
(22) Date of filing: 31.10.2012
(51) Int. Cl.: A61B 17/08, A61B 17/02

(54) **SURGICAL INCISION AND CLOSURE APPARATUS WITH INTEGRATED FORCE DISTRIBUTION**
CHIRURGISCHE INZISIONS- UND VERSCHLUSSVORRICHTUNG MIT INTEGRIERTER KRAFTVERTEILUNG
APPAREIL D'INCISION ET DE FERMETURE CHIRURGICALES AYANT UNE DISTRIBUTION DE FORCE INTÉGRÉE

(30) Priority: 01.11.2011 US 201113286757
(43) Date of publication of application: 17.09.2014
(73) Proprietor: Zipline Medical Inc., Campbell, CA 95008 (US)
(72) Inventor: BELSON, Amir, Savyon 5651420 (IL); STORNE, Eric, Menlo Park CA 94025 (US); JOHNSON, Eric T., Temecula CA 92590 (US); RAGLAND, Robert R., Temecula CA 92562 (US); BURKE, Phillip C., Pala CA 92059 (US); CLAUSON, Luke, Redwood City CA 94063 (US)
(74) Representative: Clark, Jane Anne
(86) International application number: PCT/US2012/062820
(87) International publication number: WO 2013/067024

(56) References cited:
- WO-A1-96/29013
- WO-A2-2008/060532
- US-A- 6 007 564
- US-A- 6 007 564
- US-A1- 2005 020 956
- US-A1- 2005 085 757
- US-A1- 2008 147 115
- US-A1- 2008 228 219
- US-A1- 2010 228 287
- US-A1- 2010 228 287

## Description

### BACKGROUND OF THE INVENTION

1. Field of the invention. The present invention relates generally to medical apparatus . More particularly, present invention relates to an incision closure appliance for forming and closing surgical incisions.

Surgical closure devices including an adhesive based patch with right and left panels are known. Of particular interest of the present invention, such devices are described in co-pending, commonly owned PCT application US 2010/000430 (WO 2011/043786).

As described in the PCT application, an adhesive patch is placed over a patient's skin at a site where it is desired to form a surgical incision. After the patch is placed, an incision is formed along an axial line extending through the middle of the patch. After it is formed, the incision can be opened to perform a desired procedure, and after the procedure is completed the incision may be closed by drawing the inner edges of the panels together with a clip, zipper, or other closure member.

The principal objective of such surgical closure devices is to improved healing and reduce scaring from the incision. This objective, however, has been inhibited by certain characteristics of the presently available devices. For example, the tissue edges are not always brought together along an even line, which can increase the eventual scaring. Many such closure devices do not have the ability to adjust the closure force or distance on the tissue edges, limiting the ability to slightly "pucker" tissue which has been found to reduce scaring. Other shortcomings of the available incision and wound closure devices include difficulty of use and inability to conform to tissue manipulation during subsequent surgical protocols, i.e. those devices which are sufficiently rigid to securely close the tissue are often unable to conform to the tissue movement during the surgical procedure.

A particular problem arises with self-adhesive wound closure patches when they're used beneath an adherent surgical incision drape. Such drapes are used to help maintain the sterility of a tissue surface during a surgical procedure, and the drapes may be placed over a previously positioned tissue closure patch. As the surgical incision drape has an adhesive lower surface which adheres to the tissue, the drape will adhere to an upper surface of an underlying tissue closure patch. Removal of the surgical incision drape will thus often remove or at least displace a previously placed tissue closure patch. If any significant portion of the tissue closure patch is removed or displaced, the patch will no longer be useful for closing a surgical wound.

For these reasons, it would be desirable to provide improved surgical incision closure devices and methods for their use. It would be particularly desirable to provide incision closure devices which are able to adhere to the tissue, allow formation of the incision, conform to the deformation of the tissue during a subsequent surgical procedure, and provide controlled closure of the adjacent tissue edges subsequent to the procedure. In particular, it would be desirable if the incision closure devices were able to provide for the control and the uniform distribution of closure forces on the tissue edges while causing minimum restraint or stretching of the tissue during the surgical procedure. It would be still further desirable to provide improved surgical incision closure devices and methods for their use where the devices will resist removal and dislocation when used beneath a surgical incision drape. At least some of these objectives will be met by the inventions described below.

2. Description of the Background Art. Co-pending, commonly owned PCT application US 2010/000430 as been described above. Other surgical closure devices are described in the following US Patents: 2,012,755; 3,516,409; 3,863,640; 3,933,158; 4,114,624; 3,926,193; 4,535,772; 4,676,245; 4,881,546; 4,905,694; 5,377,695; and 7,455,681; and U.S. Patent Publication Nos. 2005/0020956 and 2008/0114396. Commercial incision closure devices available from Ethicon, a division of Johnson & Johnson, under the trade name Ethizip™ temporary abdominal wound closure device.

US 2005/0085757 describes an expandable temporary abdominal closure. The closure system includes a sterile supple sheet the peripheral portions of which may be reinforced with reinforcement strips adhered to an upper surface or lower surface of the peripheral portions through adhesive or thermal bonding. A pressure sensitive adhesive may be placed on a lower surface of the sheet at the peripheral portions to assist in locating and holding these portions against the skin either independently or in conjunction with sutures or staples. The adhesive may be covered with a protective release liner removed prior to use. The sheet has a centre expandable portion which may be positioned above a wound.

### SUMMARY OF THE INVENTION

The present invention is set out in the appended claims.

Associated methods are also described herein to aid understanding the invention. These methods do not form part of the claimed invention.

Described herein are improved apparatus and methods for closing wounds, particularly wounds resulting from incisions performed during surgical procedures. The incisions would usually be formed in a patient's skin, such as through the abdomen, but in some cases could also be on internal organs, within the oral cavity, within body cavities, or alike.

The described devices and methods will present minimum disruption of or interference with the surgical procedure which is performed after the incision is made. In particular, the devices and methods will permit the opposed edges of the incised tissue to be opened, stretched, and freely deformed with minimal restraint resulting from the presence of the closure device. Once the procedure has been completed, however, the described devices and methods will provide for a uniform distribution of closure forces to draw the tissue edges together in a manner which will minimize scaring. In particular, the closure devices can draw the tissue edges together at a slightly closer spacing than initially present at the forming of
the incision in order to upwardly evert the tissue edges cause a "pucker" which can reduce scaring.

The described devices and methods will also be able to avoid or reduce disruption when a incision closure appliance is used beneath surgical incision drape which must be removed from over the closure appliance. A sacrificial cover provided over at least part of the upper surface of the closure appliance, where the sacrificial cover is held in place while the surgical incision drape is placed over the incision closure appliance. After the incision and surgical procedure have been completed, the surgical incision drape will be pulled from the patient's skin. Instead of adhering to and dislodging the tissue closure appliance, the surgical drape adheres to the sacrificial cover, and only the sacrificial cover is pulled from the patient with the drape, leaving the remainder of the incision closure appliance in place.

Described herein is an incision closure appliance comprising a base including a left panel and a right panel. Each panel has a tissue adherent lower surface, an upper surface, an inner edge, and an outer edge. The lower tissue adherent surface will typically be coated at least partially with a common tissue-adherent adhesive such as those used in surgical bandages and patches.

The incision closure appliance further includes a force distribution structure coupled to each panel (i.e. each panel will have at least one force distribution structure coupled thereto), where each force distribution structure is adapted to allow axial expansion of the panel along the inner edge while limiting lateral expansion over the entire length and axial expansion along the outer edge. By permitting axial expansion of the panel along the inner edge, the tissue edges are minimally constrained to allow the tissue to deform when stretched during the surgical procedure. Conversely, by limiting both lateral expansion and axial expansion along the outer edge, the panel will be able to apply a controlled and distributed closure force when the panels are drawn together after the surgical procedure is complete, as described in more detail below.

The incision closure appliance still further includes a closure component or assembly which attaches to the force distribution structure to draw the inner edges of the panels together after they had been adhered to the tissue on opposite sides of an incision site and the surgical procedure completed. Each panel of the base will typically comprise an at least partially elastic matrix, typically having an isotropic elasticity (i.e. the panel stretches evenly in all directions) but optionally having an anisotropic elasticity (where the matrix stretches preferentially in one direction or over a portion thereof). The elastic matrix may comprise an elastomeric membrane or sheet (for example Polyurethane sheet or Thermo Plastic Elastomers (TPE)), a woven fabric (typically woven at least partially from elastomeric filaments, threads, or fibers), a spun fabric, or the like. In certain embodiments, the elastomeric matrix may comprise a fabric woven from both elastic elements (typically threads, filaments, fibers, or the like) and having inelastic elements disposed along the outer edge and extending laterally there across in order to provide the expansion characteristics described above with respect to the force distribution structure. That is, in some cases, the force distribution structure may include or consist of inelastic elements woven or otherwise incorporated within a fabric membrane.

Typically, the force distribution structure will comprise a separate component of the incision closure appliance, for example including a spine disposed axially adjacent to the outer edge of the panel and a plurality of axially spaced-apart lateral supports disposed laterally and extending from the spine toward the inner edge of the panel. Such a "comb-like" structure will typically be formed from flexible but non-distensible materials so that the elements can flex together with the tissue deformation but will not stretch along their lengths so that they may provide dimensional stability in the lateral direction as well as along the outer edge of the panel. Examples of such materials include Nylon, Polypropylene, Polyethylene and Polycarbonate or other thermo polymers. Notably, the force distribution structure will not limit the axial stretching of the inner edge of the panel in order to provide the desired expansibility and conforms to the tissue during the surgical procedure. Such separate force distribution structures may be attached to the upper surface of the panel, or alternatively may be embedded in or laminated within the panel. Typically, the force distribution structure will not extend into or past the lower surface of the panel so that it will not interfere with adherence of the panel to the skin or other tissue.

The assembly of the base panels and the force distribution structures will typically be carried on a removable backing which covers and protects the adherent surface of the panels prior to use. The adherent backing may be removed in order to apply the base to the skin or other tissue at the site of the surgical intervention. Additionally, the right and left panels will typically be held together by removable tabs, an axial strip, or other removable covers or structures in order to hold the inner edges of the panel at a pre-determined distance or spacing as they are being adhered to the tissue. For example, removable tabs may be placed at each axial end of the base to temporarily secure the two base panels together. Alternatively, a removable strip or tape may be placed over an axial gap between the right and left panels to hold the panels in place relative to each other as the base is being adhered to the tissue surface. Such tabs or strips will typically be self-adhesive so that they may be secured to the panels and then removed by simply pulling off after the panels are properly placed. The cover, tabs, or strip may then be removed to leave the panels in place but unconnected prior to forming the surgical incision therebetween.

A first exemplary construction of the closure component or assembly comprises a right engagement member, a left engagement member, and a plurality of lateral struts holding the engagement members laterally apart by a pre-determined distance. The right engagement member is adapted to releasably engage the supports of the right panel along an inner edge thereof, and the left engagement member is adapted to releasably engage the supports of the left panel along an inner edge thereof. In the specific embodiments, at least some of the supports of the force distribution component will have cleats near their inner edges, and the engagement members will have slots which receive the cleats. After the surgical intervention is complete, the closure component may then be placed over the force distribution structure with the cleats on one side first being engaged by an engagement member and then the opposite engagement member being pulled over the cleats on the opposite side.

Alternatively, the closure component or assembly may comprise a plurality of independent lateral ties attached to at least some of the lateral supports. Such lateral ties are configured to be secured between the lateral supports, typically being fixed to one panel and being adjustably attachable to the other panel. For the exemplary embodiments, the adjustably attachable end may comprise a ratchet tightening mechanism or similar structure which allows each lateral tie to be independently adjusted at a different spacing between the right and left panels. In this way, the right and left panels may be differentially tensioned along their inner edges in order to control and optimize the forces applied to the adjacent tissue edges which are being drawn together.

Optionally, the closure appliance may further comprise a securing layer which is adapted to be placed over the assembly of the base and the closure component after the assembly has been secured over an incision on a patient's skin and the surgical procedure has been completed. A securing layer will typically have a self-adhesive lower surface which can be placed over the assembly of the base and closure component to help secure it in place and to maintain cleanliness. The securing layer may optionally have openings to permit access to the wound for observation, delivery of antiseptics, and the like.

Described herein are methods for forming an incision in tissue comprising providing an incision closure appliance as described above. The right and left panels of the appliance are adhered to the patient's skin, where the inner edges of the panels are spaced-apart by a pre-selected distance typically from 0.5 mm to 15 mm. An incision (typically linear) is formed in the tissue or skin surface between the inner edges of the panels, and the edges of the incised tissue are then separated to perform a desired surgical procedure. The inner edges of the panels can stretch and conform along with movement and deformation of the tissue edges while the outer edge and lateral extent of each panel remain dimensionally stable. After the procedure is complete, the closure component is secured to the force distribution structure to draw the inner edges of the panels back together. Optionally, the closure component has dimensions (or an adjustable inter-panel spacing) which draw the tissue edges closer together than they were immediately after the incision was formed. Such drawing together of the tissue causes the edges to evert and the tissue to "pucker" which can reduce scarring.

### BRIEF DESCRIPTION OF THE DRAWINGS

Fig. 1 is an exploded view of an incision closure appliance constructed in accordance with the principles of the present invention.
Fig. 2 is a top view of the assembly of a base and a force distribution structure which is part of the incision closure appliance.
Fig. 3 is a cross-sectional view taken along line 3-3 of Fig. 2.
Figs. 4-7 illustrate use of the incision closure appliance of the present invention for forming and closing an incision in a patient's skin.
Fig. 8 illustrates an alternative construction of a closure component for the closure appliance of the present invention.
Fig. 9 is an exploded view of a further embodiment of an incision closure appliance constructed in accordance with the principles of the present invention.
Fig 10 is an enlarged isometric view of the base and force distribution structure of the system of Fig. 9.
Figs. 11A and 11B illustrate an alternative lateral tie construction which can be used in the appliances of either Fig. 1 or Fig. 9.
Fig. 12 illustrates a sacrificial cover positioned over an incision closure appliance in accordance with the principles of the present invention.
Figs. 13A through 13E illustrate the principle of operation of the sacrificial cover illustrated in Fig. 12 when used together with a surgical incision drape.

### DETAILED DESCRIPTION

The described apparatus and methods will be used during both the formation and the closure of surgical incisions made to a patient's skin or other tissue during surgical procedures. As described hereinafter, the direction of the incision will define both "axial" and "lateral" directions as those terms are used herein. Most incisions will be made along a generally straight line which will define the axial direction. The lateral direction will generally be across the axial direction, typically but not necessarily being perpendicular or normal to the axial direction. Most incisions will be generally linear but in some cases the incisions could be curved or have other geometries. The term "axial" will then apply to the direction of the incision at any particular location, resulting in lateral directions which could also vary.

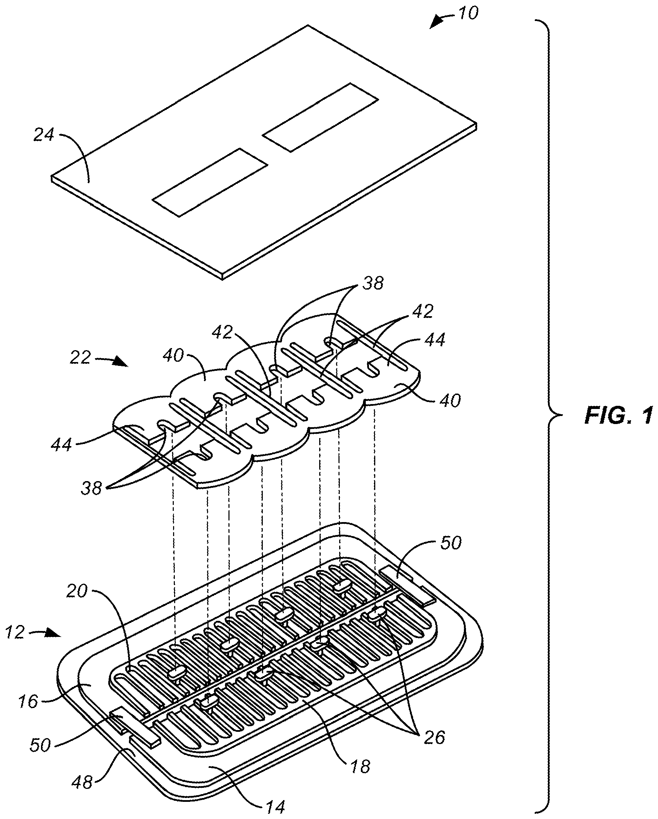

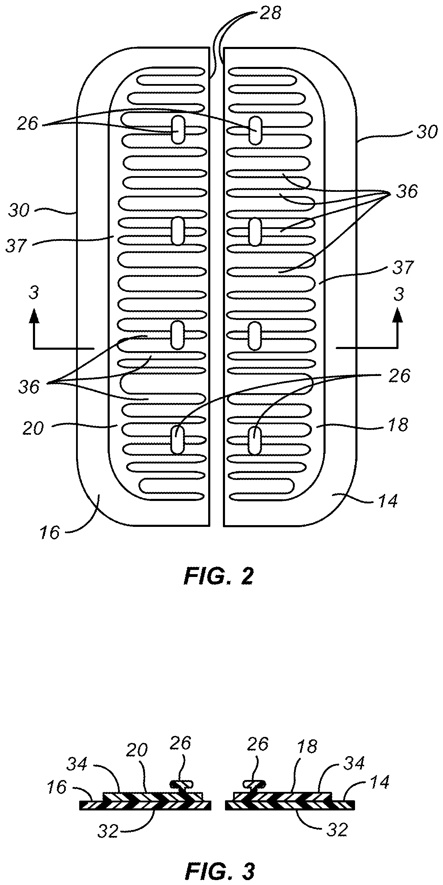

Referring now to Figs. 1-3, an incision closure appliance 10 comprises a base 12 including a right panel 14 and a left panel 16. A right force distribution structure 18 is secured to the right panel 14, typically by laminating the force distribution structure to an upper surface of the panel, and a left force distribution structure 20 is similarly attached to an upper surface of the left panel 16. The incision closure appliance further comprises a closure component 22 which is removably attachable to the right and left forced distribution structures 18 and 20 in order to close an incision, as described in more detail below, and the appliance is completed with an optional securing layer 24 which may be placed over the combined base
12 and closure component 22 after they have been secured to the patient and the incision has been closed by drawing the panels together using the closure component.

The closure component 22 is intended and adapted to draw the inner portions of the force distribution structures 18 and 20 inwardly toward each other to close a surgical incision which has been formed therebetween. In the illustrated embodiment, a plurality of cleats 26 are formed on lateral supports 36 which are held axially by spine 37 of the force distribution structures 18 and 20. The cleats 26 are received in slots 38 formed along inner edges of opposed engagement members 40 of the closure component 22. The opposed engagement members 40 are held together by lateral struts 42 so that the engagement members are held at a fixed, laterally spaced-apart distance (in other embodiments the spaced-apart distance may be adjustable). The slots 38 are preferably formed on flexible tab-like structures 44 which allow the slots to be pulled upwardly over the corresponding cleats in order to secure the closure component 22 over the force distribution structures 18 and 20.

The lower surfaces 32 of each panel 18 and 20 will typically be covered with a pressure-responsive adhesive, where the adhesive is initially covered with a protective layer 48 which may be peeled away immediately prior to use. Additionally, pull-away tabs 50 or other similar structures may be provided in order to hold the right and left panels 14 and 16 together at a pre-determined spaced-apart distance after the layer 48 has been removed but prior to adhering the panels to a patient's skin or other tissue surface. It is important that the distance between the inner edges 28 of each panel 14 and 16 be maintained as close as possible to the original target spacing so that the tissue edges, when closed by the closure component 22, will be precisely brought together, typically with a slight eversion.

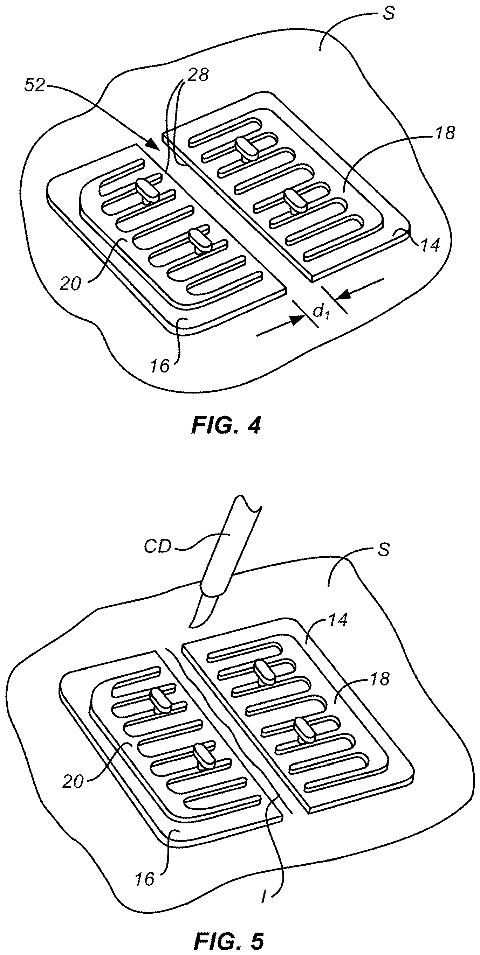

Referring now to Figs. 4 through 7, a protocol for both forming an incision and subsequently closing the incision will
be described. Initially, the right and left panels 14 and 16 are placed on the patient's skin followed by reference letter S, as shown in Fig. 4. The panels 14 and 16 are applied by first pulling away the protective layer 18 and placing the panels onto the tissue, after which time the tabs 50 may be removed, leaving an incision path 52 defined between the inner edges 28. The spacing of the inner edges 28 will be selected to provide a fixed, pre-determined distance d₁.

After the right and left panels 14 and 16 are in place, an incision I can be formed in the space between the panels using a scalpel or other surgical cutting device CD, as shown in Fig. 5.

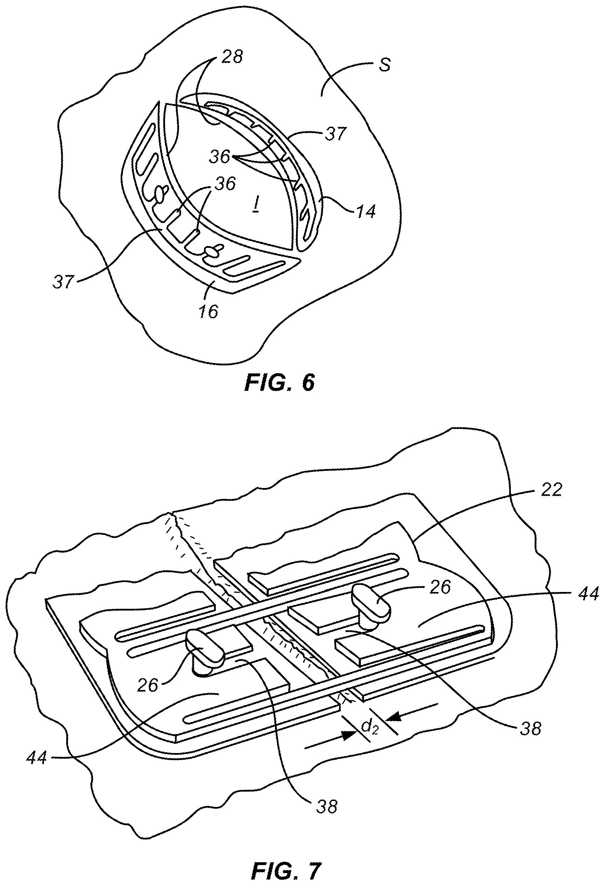

After the incision I is made, a surgical procedure may be performed by opening the inner edges of the incision which in turn deforms the inner edges 28 of the right and left panels 14 and 16, as shown in Fig. 6. As the inner most ends of the supports 36 are not connected, they are free to separate and allow the elastic matrix of the right and left panels 14 and 16 to expand, as clearly in Fig. 6. The dimensional stability of the remainder of the panels, however, will be preserved by the lateral supports 36 as well as the axial spines 37 which do not elongate under the influence of the force applied by stretching opening the incision.

After the surgical procedure is complete, the closure component 22 will be secured over the force distribution structures 18 and 20, as illustrated in Fig. 7. In particular, the slots 38 in the tab-like structures 44 are engaged over opposed cleats 26 in order to draw opposed edges of the panels as well as of the tissue incision together. By properly spacing the depth of the slots 38, the closure component 22 can be tailored so that the panels 14 and 16 are brought together by a pre-selected distance d₂. Typically, the distance d₂ will be less than the initial separation d₁ so that the inner edges of the tissue are brought together to cause the tissue edges along the incision to slightly evert (pucker upwardly) which can improve healing and reduce scarring.

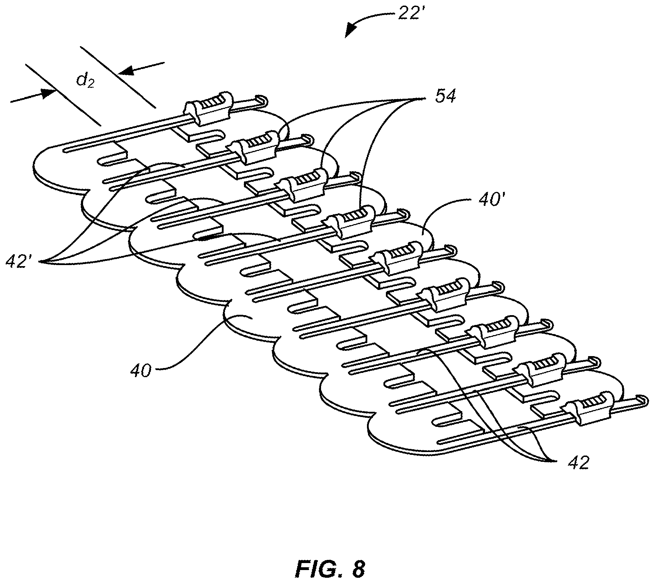

Optionally, as shown in Fig. 8, a closure component 22' may include engagement members 40', where one end of each lateral strut 42' is joined by an adjustable clasp or other mechanism 54 so that the distance between the inner edges of the opposed engagement members 40' can be adjusted in order to increase or lessen the distance d₂ therebetween.

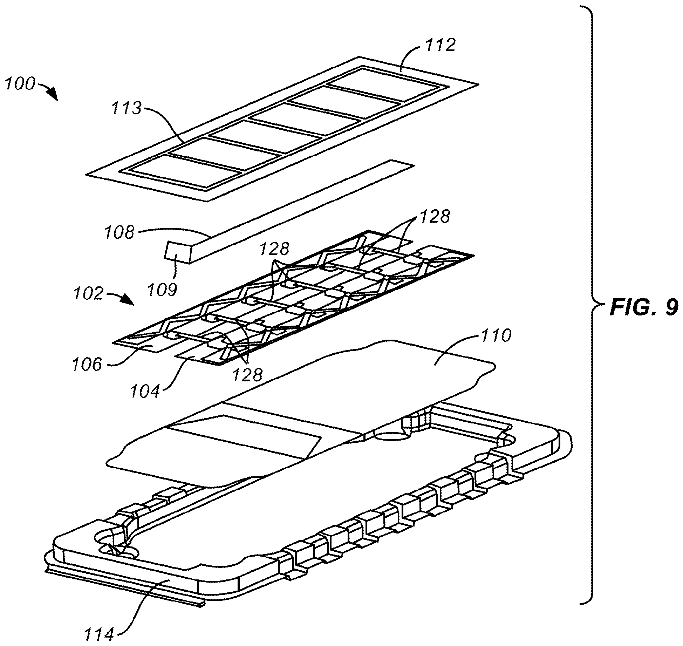

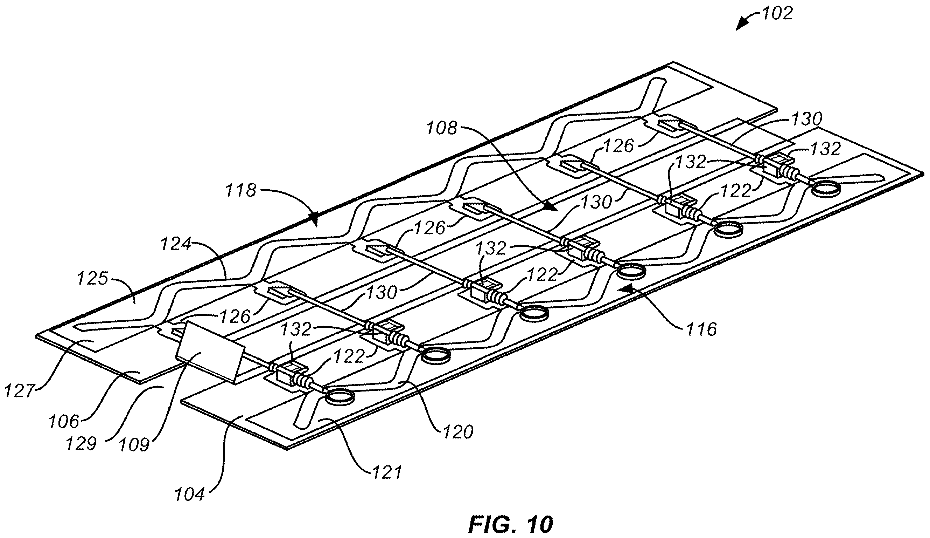

An alternative embodiment 100 of the incision closure appliance of the present invention is illustrated in Figs. 9 and 10. The appliance 100 includes a base 102 having a right panel 104 and a left panel 106. A positioning or alignment strip 108 is provided to secure the inner edges of each panel together, as shown best in Fig. 10 and includes an end tab 109 that allows the user to pull the strip from the panels 104 and 106 after the panels have been put in place on a tissue surface.

The incision closure appliance 100 further includes a backing 110 having an end which may be partially folded back to expose an underlying adhesive backing on the panels and allow that end of the base 102 to be adhered to the tissue while the remainder of the base
is still covered by the backing. A securing layer 112 which includes a reinforcement frame 113 is provided for placement over the right panel 104 and left panel 106 after the base
102 has been closed over an incision, generally is described in connection with the previous embodiment. Usually, a holding tray 114 will be provided for maintain the components of the appliance together in a sterilized condition where the tray 114 will be covered with conventional medical packaging cover.

As illustrated in Figs. 9 and 10, a right force distribution structure 116 and a left force distribution structure 118 are provided on the upper surfaces of the right panel 104 and the left panel 106, respectively. The right force distribution structure 116 includes a right axial spine 120 and a plurality of lateral supports 122. Typically, the right axial spine 120 comprises a serpentine or zig-zag number which is embedded in or laminated to a base strip 121. The serpentine axial spine 120 would typically be formed from a flexible, resilient plastic, typically a hard plastic, while the base strip 121 will be comprised of a polyurethane or similar plastic layer. The lower surface of the polyurethane layer will be covered with a hydrocolloid layer for tissue adhesion. The structure of the left forced distribution structure 118 will be the same including a left axial spine 124, left lateral supports 126, and a left base strip 127.

The incision closure appliance 100 will include a closure mechanism comprising a plurality of lateral tie assemblies 128 as shown on Fig. 9. As best seen in Fig. 10, each lateral tie assembly 128 will include a rod which is secured at one end to the left lateral support 126 and a ratchet mechanism 132 which is secured to the right lateral support 122. Each rod 130 will usually be aligned with the axis of the left panel 106 parties so that a gap 129 between the right panel 104 and left panel 106 will be left open so that an incision can be made there between. After the incision is made, each rod 130 will be pulled over to the associated ratchet 132 on the right panel 104. A series of ratchet rings on each rod will be pulled into the associated ratchet mechanism 132, and the rod then pulled laterally until the desired closing tension is applied at that point along the base 102. It is a particular advantage that each of the lateral tie assemblies 128 may be individually adjusted to supply the desired closing tension across the tissue along the length of the incision being closed. Once the desired closing tension has been provided along the entire incision, the securing layer 112 may be placed over base 102 to hold the appliance and tissue in place.

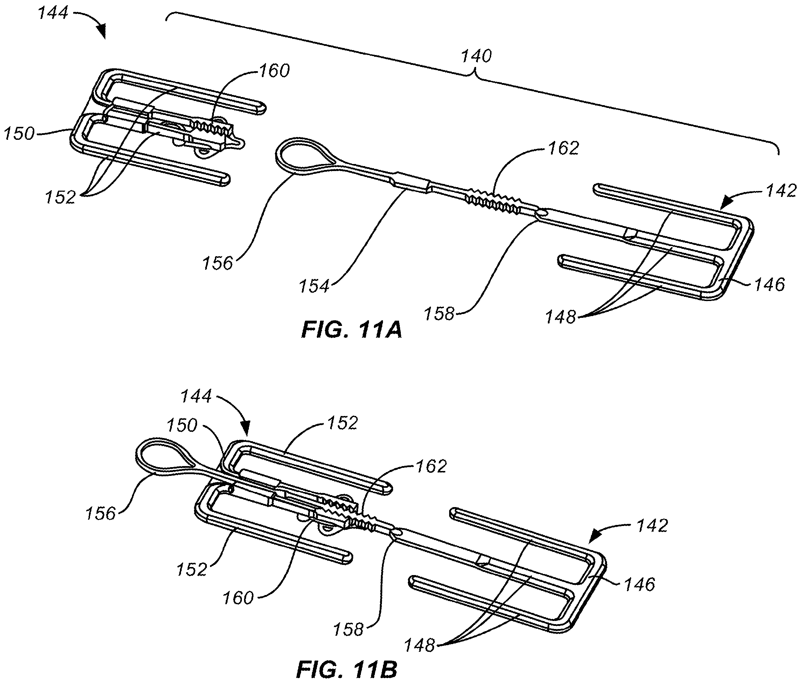

Referring now to Figs. 11A and 11B, an alternative design for the lateral tie assemblies 140 of the present invention is illustrated. These lateral tie assemblies 140 may be utilized with either of the incision closure appliances 10 or 100 described previously. Each lateral tie assembly 140 includes a right force distribution structure 142 and a left force distribution structure 144. The right force distribution structure includes a right spine 146 and a plurality of lateral supports 148. Although three are shown, it will be appreciated that four, five, six or more lateral supports could be included. The left force distribution structure 144 similarly includes a left spine 150 and a plurality of left lateral supports 152. To provide closure, the right force distribution structure 142 includes a rod 154 which extends from the center lateral support 1 48. Typically, the rod 154 is joined to the support by a live or passive joint 158. A pull loop 156 is provided at the free end of the rod 154, and a plurality of ratchet teeth 162 are provided along the midsection of the rod 154.

The left force distribution structure 144 includes a ratchet mechanism 160 adapted to receive the teeth 162 on the rod 154 of the right force distribution structure. In this way, the rod 154 can be lowered into the ratchet 160 to engage teeth 162, allowing the rod to be pushed forward in order to draw the right and left force distribution structures 142 and 144 together in order to apply tension to the right and left panels.

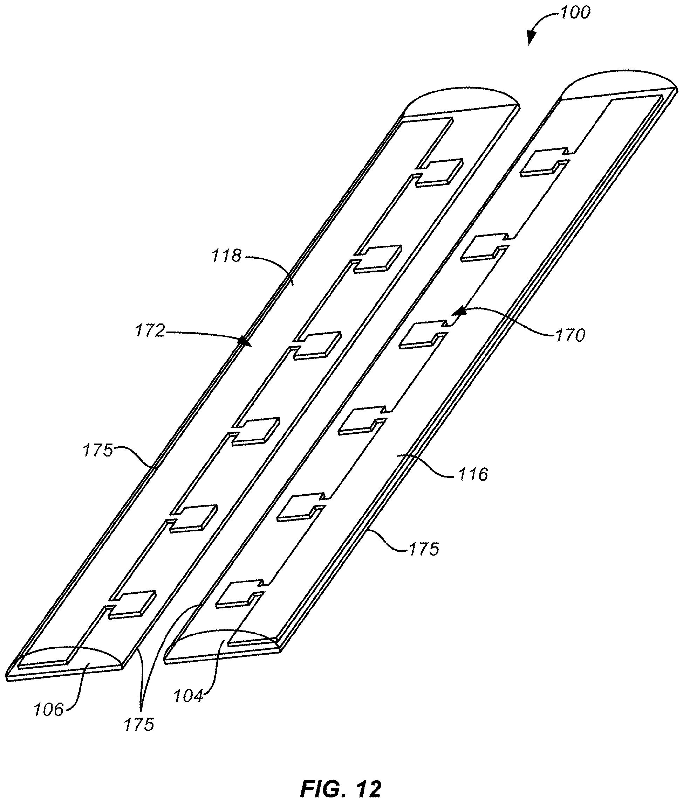

As illustrated in Fig. 12, a further aspect of the present invention is illustrated. The incision closure appliance 100 is illustrated schematically with only the right and left panels 104 and 106 and the right and left force distribution structures 116 and 118 being illustrated. The remaining system components are not shown for ease of illustration.

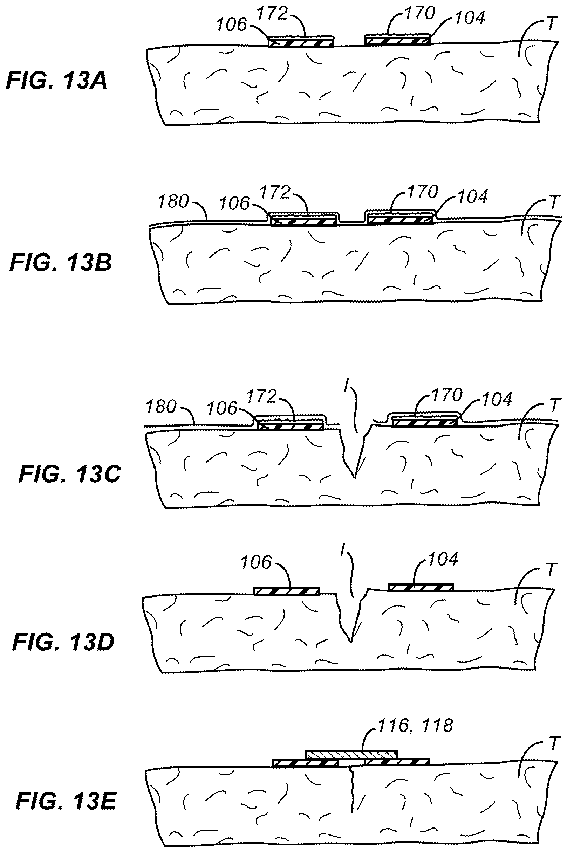

The right panel 104 is covered by a right sacrificial cover 170 and the left panel 106 is covered by a left sacrificial cover 172. Each cover 170 and 172 is detachably secured along each edge of the associated base panel so that the covers remain in place during normal handling and placement of the incision closure appliance 100 over the tissue surface to be incised. The use and purpose of these sacrificial covers 170 and 172 is described with reference to Figs. 13A and 13E.

Fig. 13A illustrates the right and left panels 104 and 106 in place on a tissue surface T prior to an incision being made. The right panel 104 is covered by right sacrificial cover 170 and the left panel 106 is covered by left sacrificial cover 172. As is common in many surgeries, an adherent surgical incision drape 180 is placed over the tissue surface T. Any conventional drape may be used such as the Ioban™ antimicrobial incise drape, available from 3M, St. Paul, Minnesota.

After the incision drape 180 is in place over the incision closure appliance, a surgical incision I may be made for performing a desired surgical intervention. As can be seen, the incision I will cut through the surgical drape 180 between the right and left panels 104 and 106, respectively. After the surgical procedure is completed, the surgical drape 180 will be removed from the tissue surface T. As the surgical drape has a lower adherent surface, prior to the present invention, removal of the drape might have displaced either or both of the right panel 104 and left panel 106. Presence of the sacrificial covers 170 and 172, however, prevents such displacement. Removal of the surgical drape 180 will remove the sacrificial cover 170 and 172, but as each of these covers is configured to break off with a relatively low separation force, removal of the sacrificial covers will not cause the underlying panels 104 or 106 to be displaced. Thus, the panels 104 and 106 will be left in place, as shown in Fig. 13D, and the force distribution structures 116 and 118 can be used as described previously for closing the panels together to close the incision as shown in Fig. 13E.

## Claims

1. An incision closure appliance comprising:
a base (12; 102) including a left panel (16; 106) and a right panel (14; 104), each panel having a tissue adherent lower surface, an upper surface, an inner edge (28), and an outer edge;
left and right force distribution structures (20 and 18; 116 and 118) coupled to the left and right panels, respectively, wherein each force distribution structure comprises an axial spine (37; 120 and 124) and lateral supports (36; 122 and 126), such that opening of the inner edges of an incision between the panels deforms the inner edges of the right and left panels, the dimensional stability of the remainder of the panels being preserved by the lateral supports as well as the axial spines, wherein the force distribution structures (20 and 18; 116 and 118) are formed from flexible but not-distensible materials; and
a closure assembly (22; 22'; 100) securable to the left and right panels to draw the inner edges of the panels together.

2. An incision closure appliance as in claim 1, wherein each panel (16; 14; 106; 104) of the base comprises an elastic matrix, wherein the elastic matrix preferably comprises an elastomeric membrane, a woven fabric, or a spun fabric.

3. An incision closure appliance as in claim 2, wherein the elastic matrix comprises a fabric woven from elastic elements and having inelastic elements along the outer edge and extending laterally thereacross.

4. An incision closure appliance as in any one of the preceding claims, wherein the spine (37; 120 and 124) and lateral supports (36; 122 and 126) are formed from flexible, non-distensible materials.

5. An incision closure appliance as in claim 4, wherein the spine and supports are formed integrally as a comb-like structure.

6. An incision closure appliance as in claim 4, wherein the force distribution structures (20 and 18; 116 and 118) are embedded in or laminated to the upper surface of each panel.

7. An incision closure appliance as in any one of the preceding claims, further comprising a removable space maintainer configured to hold the right and left panels (14; 104 and 16; 106) at a fixed distance while they are being adhered to tissue.

8. An incision closure appliance as in claim 7, wherein the removable space maintainer comprises a pair of tabs (50) which are removably placed at each axial end of the base or wherein the removable space maintainer comprises a strip (108) which is removably placed over an axial gap between the right and left panels.

9. An incision closure appliance as in any one of the preceding claim wherein the closure assembly comprises an adjustable inter-panel spacing.

10. An incision closure appliance as in any one of claims 1 to 8, wherein the closure assembly comprises a right engagement member (40; 40'), a left engagement member (40) and a plurality of lateral struts (42) holding the engagement members laterally apart by a predefined distance, wherein the right engagement member (40; 40') is adapted to releasably engage the supports of the right panel (14; 104) along the inner edge and the left engagement member (40; 40') is adapted to releasably engage the supports of the left panel (16; 106) along the inner edge.

11. An incision closure appliance as in claim 9, wherein at least some of the lateral supports (36; 122) have cleats (26) near the inner edge and the engagement members (40; 40') have slots (38) which receive the cleats (26) or wherein the lateral struts (42) are adjustably connected to at least one of the engagement members (40; 40') to permit adjustment of the predefined distance.

12. An incision closure appliance as in any one of claims 1 to 8, wherein the closure assembly comprises a plurality of independent lateral ties (128) attached to at least some of the lateral supports (122 and 126), wherein the lateral ties are configured to be secured between lateral supports.

13. An incision closure appliance as in claim 12, wherein the independent lateral ties (128) each have one end fixed to a panel and a second end adjustable attached to the other panel, wherein the second end preferably comprises a ratchet tightening mechanism.

14. An incision closure appliance as in any one of the preceding claims, further comprising a securing layer (112) adapted to be placed over an assembly of the base and the closure assembly after the assembly has been secured over an incision on a patient's skin.

15. An incision closure appliance as in claim 14, wherein the securing layer (112) has lower self-adhesive surface.

16. An incision closure appliance as in any one of the preceding claims, further comprising a sacrificial cover (170; 172) over at least a portion of an upper surface of the base, said sacrificial cover configured to lie between the upper surface of the base (12; 102) and a lower surface of an overlying adherent surgical incision drape and to separate from the base when the drape is pulled from over the base wherein the base can remain adhered to the tissue as the drape is removed.

## Patentansprüche

1. Inzisionsverschlussvorrichtung, die Folgendes umfasst:
eine Basis (12; 102) mit einem linken Paneel (16; 106) und einem rechten Paneel (14; 104), wobei jedes Paneel eine gewebeanhaftende Unterseite, eine Oberseite, einen inneren Rand (28) und einen äußeren Rand hat;
linke und rechte Kraftverteilungsstrukturen (20 und 18; 116 und 118), die mit dem linken bzw. rechten Paneel verbunden sind, wobei jede Kraftverteilungsstruktur eine axiales Rückgrat (37; 120 und 124) und laterale Stützen (36; 122 und 126) umfasst, so dass durch Öffnen der inneren Ränder einer Inzision zwischen den Paneelen die inneren Ränder des rechten und linken Paneels verformt werden, wobei die Dimensionsstabilität des restlichen Teils der Paneelen durch die lateralen Stützen sowie die axialen Rückgrate erhalten bleibt, wobei die Kraftverteilungsstrukturen (20 und 18; 116 und 118) aus flexiblen, aber nicht dehnbaren Materialien gebildet sind; und
eine Verschlussanordnung (22; 22'; 100), die am linken und rechten Paneel befestigt werden kann, um die inneren Ränder der Paneelen zusammenzuziehen.

2. Inzisionsverschlussvorrichtung nach Anspruch 1, wobei jedes Paneel (16; 14; 106; 104) der Basis eine elastische Matrix umfasst, wobei die elastische Matrix vorzugsweise eine elastomere Membran, einen gewebten Stoff oder einen gesponnenen Stoff umfasst.

3. Inzisionsverschlussvorrichtung nach Anspruch 2, wobei die elastische Matrix einen aus elastischen Elementen gewebten Stoff umfasst, der unelastische Elemente entlang des äußeren Rands hat, die sich seitlich darüber erstrecken.

4. Inzisionsverschlussvorrichtung nach einem der vorherigen Ansprüche, wobei das Rückgrat (37; 120 und 124) und die lateralen Stützen (36; 122 und 126) aus flexiblen, nicht dehnbaren Materialien gebildet sind.

5. Inzisionsverschlussvorrichtung nach Anspruch 4, wobei das Rückgrat und die Stützen einstückig als kammartige Struktur ausgebildet sind.

6. Inzisionsverschlussvorrichtung nach Anspruch 4, wobei die Kraftverteilungsstrukturen (20 und 18; 116 und 118) in die Oberseite jedes Paneels eingebettet oder darauf laminiert sind.

7. Inzisionsverschlussvorrichtung nach einem der vorherigen Ansprüche, die ferner einen entfernbaren Platzhalter umfasst, der so konfiguriert ist, dass er das rechte und linke Paneel (14; 104 und 16; 106) in einem festen Abstand hält, während sie auf Gewebe geklebt werden.

8. Inzisionsverschlussvorrichtung nach Anspruch 7, wobei der entfernbare Platzhalter ein Paar Zungen (50) umfasst, die entfernbar an jedem axialen Ende der Basis platziert sind, oder wobei der entfernbare Platzhalter einen Streifen (108) umfasst, der entfernbar über einem axialen Spalt zwischen dem rechten und linken Paneel platziert ist.

9. Inzisionsverschlussvorrichtung nach einem vorherigen Anspruch, wobei die Verschlussanordnung einen einstellbaren Zwischenpaneelabstand umfasst.

10. Inzisionsverschlussvorrichtung nach einem der Ansprüche 1 bis 8, wobei die Verschlussanordnung ein rechtes Eingriffselement (40; 40'), ein linkes Eingriffselement (40) und mehrere laterale Streben (42) umfasst, die die Eingriffselemente lateral um einen vordefinierten Abstand auseinander halten, wobei das rechte Eingriffselement (40; 40') so gestaltet ist, dass es lösbar in die Stützen des rechten Paneels (14; 104) entlang des inneren Rands eingreift und das linke Eingriffselement (40; 40') so gestaltet ist, dass es lösbar in die Stützen des linken Paneels (16; 106) entlang des inneren Rands eingreift.

11. Inzisionsverschlussvorrichtung nach Anspruch 9, wobei wenigstens einige der lateralen Stützen (36; 122) Stollen (26) nahe des inneren Rands haben und die Eingriffselemente (40; 40') Schlitze (38) haben, die die Stollen (26) aufnehmen, oder wobei die lateralen Streben (42) einstellbar mit wenigstens einem der Eingriffselemente (40; 40') verbunden sind, um eine Einstellung des vordefinierten Abstands zu ermöglichen.

12. Inzisionsverschlussvorrichtung nach einem der Ansprüche 1 bis 8, wobei die Verschlussanordnung mehrere unabhängige laterale Verbindungsstücke (128) umfasst, die an wenigstens einigen der lateralen Stützen (122 und 126) angebracht sind, wobei die lateralen Verbindungsstücke so konfiguriert sind, dass sie zwischen lateralen Stützen befestigt sind.

13. Inzisionsverschlussvorrichtung nach Anspruch 12, wobei ein Ende der unabhängigen lateralen Verbindungsstücke (128) jeweils an einem Paneel fixiert ist und ein zweites Ende an dem anderen Paneel einstellbar angebracht ist, wobei das zweite Ende vorzugsweise einen Ratschenspannmechanismus umfasst.

14. Inzisionsverschlussvorrichtung nach einem der vorherigen Ansprüche, die ferner eine Befestigungslage (112) umfasst, die so gestaltet ist, dass sie über einer Anordnung der Basis und der Verschlussanordnung platziert wird, nachdem die Anordnung über einer Inzision auf der Haut eines Patienten befestigt wurde.

15. Inzisionsverschlussvorrichtung nach Anspruch 14, wobei die Befestigungslage (112) eine selbstklebende Unterseite hat.

16. Inzisionsverschlussvorrichtung nach einem der vorherigen Ansprüche, die ferner eine Opferabdeckung (170; 172) über wenigstens einem Abschnitt einer Oberseite der Basis hat, wobei die genannte Opferabdeckung so konfiguriert ist, dass sie zwischen der Oberseite der Basis (12; 102) und einer Unterseite eines darüberliegenden anhaftenden, chirurgischen Inzisionstuchs liegt und sich von der Basis trennt, wenn das Tuch von der Basis gezogen wird, wobei die Basis am Gewebe haften bleiben kann, während das Tuch entfernt wird.

## Revendications

1. Appareil de fermeture d'incision comprenant :
une base (12 ; 102) incluant un panneau gauche (16 ; 106) et un panneau droit (14 ; 104), chaque panneau ayant une surface inférieure adhérant au tissu, une surface supérieure, un bord interne (28), et un bord externe ;
des structures de distribution de force droite et gauche (20 et 18 ; 116 et 118) couplée aux panneaux gauche et droit, respectivement, dans lequel chaque structure de distribution de force comprend une arête dorsale axiale (37 ; 120 et 124) et des supports latéraux (36 ; 122 et 126), de sorte que l'ouverture des bords internes d'une incision entre les panneaux déforme les bords internes des panneaux droit et gauche, la stabilité dimensionnelle du reste des panneaux étant préservée par les supports latéraux ainsi que les arêtes dorsales axiales, dans lequel les structures de distribution de force (20 et 18 ; 116 et 118) sont formées à partir de matériaux souples mais non dilatables ; et
un ensemble de fermeture (22 ; 22' ; 100) pouvant être fixé aux panneaux gauche et droit pour tirer les bords internes des panneaux l'un vers l'autre.

2. Appareil de fermeture d'incision selon la revendication 1, dans lequel chaque panneau (16 ; 14 ; 106 ; 104) de la base comprend une matrice élastique, dans lequel la matrice élastique comprend préférablement une membrane élastomère, un tissu tissé, ou un tissu filé.

3. Appareil de fermeture d'incision selon la revendication 2, dans lequel la matrice élastique comprend un tissu tissé à partir d'éléments élastiques et ayant des éléments inélastiques le long du bord externe et s'étendant latéralement à travers celle-ci.

4. Appareil de fermeture d'incision selon l'une quelconque des revendications précédentes, dans lequel l'arête dorsale (37 ; 120 et 124) et les supports latéraux (36 ; 122 et 126) sont formés à partir de matériaux non dilatables, souples.

5. Appareil de fermeture d'incision selon la revendication 4, dans lequel l'arête dorsale et les supports sont formés d'un seul tenant comme une structure en forme de peigne.

6. Appareil de fermeture d'incision selon la revendication 4, dans lequel les structures de distribution de force (20 et 18 ; 116 et 118) sont incrustées dans ou laminées sur la surface supérieure de chaque panneau.

7. Appareil de fermeture d'incision selon l'une quelconque des revendications précédentes, comprenant en outre un mainteneur d'espace amovible configuré pour tenir les panneaux droit et gauche (14 ; 104 et 16 ; 106) à une distance fixe pendant qu'ils sont amenés à adhérer au tissu.

8. Appareil de fermeture d'incision selon la revendication 7, dans lequel le mainteneur d'espace amovible comprend une paire de languettes (50) qui sont placées de manière amovible au niveau de chaque extrémité axiale de la base ou dans lequel le mainteneur d'espace amovible comprend une bande (108) qui est placée de manière amovible par-dessus un écart axial entre les panneaux droit et gauche.

9. Appareil de fermeture d'incision selon l'une quelconque des revendications précédentes dans lequel l'ensemble de fermeture comprend un espacement entre panneaux ajustable.

10. Appareil de fermeture d'incision selon l'une quelconque des revendications 1 à 8, dans lequel l'ensemble de fermeture comprend un élément d'entrée en prise droit (40 ; 40'), un élément d'entrée en prise gauche (40) et une pluralité de montants latéraux (42) tenant les éléments d'entrée en prise à l'écart l'un de l'autre latéralement sur une distance prédéfinie, dans lequel l'élément d'entrée en prise droit (40 ; 40') est conçu pour entrer en prise de manière libérable avec les supports du panneau droit (14 ; 104) le long du bord interne et l'élément d'entrée en prise gauche (40 ; 40') est conçu pour entrer en prise de manière libérable avec les supports du panneau gauche (16 ; 106) le long du bord interne.

11. Appareil de fermeture d'incision selon la revendication 9, dans lequel au moins quelques-uns des supports latéraux (36 ; 122) ont des taquets (26) près du bord interne et les éléments d'entrée en prise (40 ; 40') ont des fentes (38) qui reçoivent les taquets (26) ou dans lequel les montants latéraux (42) sont connectés de manière ajustable à au moins l'un des éléments d'entrée en prise (40 ; 40') pour permettre l'ajustement de la distance prédéfinie.

12. Appareil de fermeture d'incision selon l'une quelconque des revendications 1 à 8, dans lequel l'ensemble de fermeture comprend une pluralité de liens latéraux indépendants (128) attachés à au moins quelques-uns des supports latéraux (122 et 126), dans lequel les liens latéraux sont configurés pour être fixés entre les supports latéraux.

13. Appareil de fermeture d'incision selon la revendication 12, dans lequel les liens latéraux indépendants (128) ont chacun une extrémité fixée à un panneau et une deuxième extrémité ajustable attachée à l'autre panneau, dans lequel la deuxième extrémité comprend préférablement un mécanisme de serrage à cliquet.

14. Appareil de fermeture d'incision selon l'une quelconque des revendications précédentes, comprenant en outre une couche de fixation (112) conçue pour être placée par-dessus un ensemble de la base et de l'ensemble de fermeture après que l'ensemble a été fixé par-dessus une incision sur la peau d'un patient.

15. Appareil de fermeture d'incision selon la revendication 14, dans lequel la couche de fixation (112) a une surface inférieure auto-adhésive.

16. Appareil de fermeture d'incision selon l'une quelconque des revendications précédentes, comprenant en outre une couverture sacrificielle (170 ; 172) par-dessus au moins une partie d'une surface supérieure de la base, ladite couverture sacrificielle configurée pour reposer entre la surface supérieure de la base (12 ; 102) et une surface inférieure d'un champ opératoire pour incision adhésif sus-jacent et pour se séparer de la base quand le champ est tiré de sur la base dans lequel la base peut continuer d'adhérer au tissu quand le champ est retiré.
